# EUROPEAN PATENT APPLICATION

(11) **EP 4 678 231 A1**
(43) Date of publication of application: **14.01.2026**
(21) Application number: 25187610.8
(22) Date of filing: 04.07.2025
(51) Int. Cl.: A61N 7/00, A61N 2/06

(54) **A NON-INVASIVE MAGNETO-ACOUSTIC APPARATUS FOR HEALTHY TISSUE HEALING, REGENERATION AND CANCER REGRESSION**

(30) Priority: 11.07.2024 IN 202411053207
(71) Applicant: Council Of Scientific & Industrial Research, New Delhi 110 001 (IN)
(72) Inventor: BALLA, Vamsi Krishna, 700032 Kolkata, West Bengal (IN); BODHAK, Subhadip, 700032 Kolkata, West Bengal (IN); SENGUPTA, Somoshree, 700032 Kolkata, West Bengal (IN); SARKAR, Siddik, 700032 Kolkata, West Bengal (IN); RAHAMAN, Shaikh Hasanur, 700032 Kolkata, West Bengal (IN); SHAW, Sweta, 700032 Kolkata, West Bengal (IN); SAHA, Debolina, 700032 Kolkata, West Bengal (IN)
(74) Representative: Cabinet Chaillot

(57) **Abstract**

The present invention relates to a non-invasive magneto-acoustic apparatus for healthy tissue healing and cancer regression comprises at least one pulsed ultrasound unit (LIPUS), at least one permanent magnet, at least one module housing, at least one permanent magnets, at least one control system for controlling pulsed ultrasound unit to energize the unit with desired pulse duration, intensity and active/ON time. The invention is to synchronously uses the low intensity pulsed ultrasound (LIPUS) and static magnetic field (SMF), to selectively activate cellular activities specific to cartilage, bone, skin leading to accelerated tissue healing, regeneration. The invention apparatus is to inhibit cancer cellular activities specific to cancers of breast, skin, bone leading to enhanced cancer regression, while leaving the healthy cells unaffected using magneto-acoustic apparatus appropriately. The apparatus exhibit no toxicity to healthy cells and can reduce overall treatment time and costs due to accelerated tissue repair and tumor regression.

## Description

### FIELD OF INVENTION:

The present invention relates to non-invasive magneto-acoustic apparatus for degenerated, injured tissues and diseased tissues.

In particular, the invention is related to an apparatus that synchronously uses low intensity pulsed ultrasound (LIPUS) and static magnetic field (SMF) to generate in situ electro-mechanical stimuli thereby modulating cellular activities for accelerated tissue healing, regeneration of degenerated, injured tissues (cartilage, bone, skin) and inhibit rapidly proliferating diseased tissue cells (breast, skin, bone) for efficient cancer treatment.

### BACKGROUND OF INVENTION:

Reference may be made to US9987140B2 (Dynamic force generation for bone repair) wherein, an implantable orthopaedic device delivers dynamic forces to a desired remote bone region in need of repair. This device promotes fracture healing, treats osteoporotic or other poor-quality bone, and promotes vertebral fusion in conjunction with a spinal fusion procedure. No use of ultrasound and/or static magnetic fields, which are key embodiments of the present invention which relates to a magneto-acoustic apparatus that synchronously uses LIPUS and SMF to generate in situ electro-mechanical stimuli thereby modulating cellular activities for accelerated tissue healing/regeneration of degenerated/injured tissues (cartilage/bone/skin) and treating diseased tissues (breast/skin/bone cancer).

Reference may be made to US11618874B2 (Methods for modulating osteochondral development using bioelectrical stimulation) wherein, devices and stimulus waveforms (unique pulsed electromagnetic field (PEMF) wave patterns) are provided to differentially modulate the behavior of osteoblasts, chondrocytes and other connective tissue cells to promote proliferation, differentiation, matrix formation or mineralization for in vitro or in vivo applications. The methods and devices described are useful in promoting repair of bone fractures, cartilage and connective tissue repair as well as for engineering tissue for transplantation. No use of ultrasound and/or static magnetic fields, which are key embodiments of the present invention which relates to a magneto-acoustic apparatus that synchronously uses LIPUS and SMF.

Reference may be made to US9486638B2 (Thermally assisted pulsed electro-magnetic field stimulation device and method for treatment of osteoarthritis) wherein, a method, apparatus and a system for thermally-assisted (38-42°C) pulsed electromagnetic field stimulation for treatment of osteoarthritis are disclosed. At elevated temperatures the healing effect of PEMF stimulation on the cartilage is maximized and overall efficiency of the treatment is improved. Here also no use of ultrasound and/or static magnetic fields, which are key embodiments of the present invention related to a magneto-acoustic apparatus.

Reference may be made to WO2007149811 (Apparatus and method for stimulation of biological tissue) wherein, the apparatus comprises an electric source capable of generating electric field across a region of tissue and means for altering the permittivity of the tissue (through chemical, optical, mechanical, thermal, or electromagnetic source) relative to the electric field, whereby a displacement current is generated. Use pulsed electrical fields, implanted electrodes/device. No mention about the use of ultrasound and/or static magnetic fields as disclosed in the present invention related to magneto-acoustic apparatus.

Reference may be made to US-20060106424-A1 (Ultrasound Device and Method of Use) wherein, a method of treating bacterial and/or fungal infections by stimulating a cell, cells or tissue with ultrasound is disclosed. They used low intensity pulsed ultrasound 1.3-2 MHz, pulses 100-1000 MHz, pulse duration 10-2000 microseconds, up to 100 mW/cm2 for the stated treatment. However, the method does not include the use of static magnetic fields and moreover for treatment of tissue healing/regeneration and cancer treatment, which are part of present invention related to magneto-acoustic apparatus.

Reference may be made to US7722539B2 (Treatment of unwanted tissue by the selective destruction of vasculature providing nutrients to the tissue) wherein, a method for using high intensity focused ultrasound (HIFU) to treat undesired tissue by damaging selected vascular regions to affect a viability of the undesired tissue, without directly targeting the undesired tissue with the HIFU. Blood flow in selected portions of the vasculature occluded by selectively treating specific portions of the vasculature with HIFU. The occlusion denies undesired tissue the nutrients and oxygen provided by blood flow, causing necrosis in the undesired tissue. But the invention do not use low intensity pulsed ultrasound and/or static magnetic fields for treatment and for tissue healing/regeneration or cancer regression, which are primary embodiments of present invention related to magneto-acoustic apparatus. Reference may be made to US11607543B2 (Delivering tumor treating fields (TTFields) using implantable transducer arrays) wherein, tumor treating fields (TTFields) can be delivered by implanting a plurality of sets of implantable electrode elements within a person's body. Temperature sensors positioned to measure the temperature at the electrode elements are also implanted, along with a circuit that collects temperature measurements from the temperature sensors. In some embodiments, an AC voltage generator configured to apply an AC voltage across the plurality of sets of electrode elements is also implanted within the person's body. Whereas the present invention relates to a magneto-acoustic apparatus that synchronously uses LIPUS and SMF, which are absent in US11607543B2**.**

Reference may be made to WO2015077005A1 (Method of treating skin cancer using low intensity ultrasound) wherein, the present disclosure relates to methods of treating skin cancer using low intensity ultrasound (27 kHz to 2.2 MHz, 0.1 to 5 W/cm2). Low intensity ultrasound can selectively kill skin cancer cells. In some embodiments, the low intensity ultrasound can be continuous and focused. However, the use of SMF in combination with LIPUS and for tissue healing/regeneration or breast cancer treatment is absent.

Reference may be made to US6926659B1 (Magnetic field enhancement of tumor treatment), wherein, a method of treating a tumor, comprising creating an elevated concentration of free radicals in said tumor and creating a magnetic field that travers said tumor and that inhibits the recombination of said free radicals in said tumor, thereby increasing the rate of apoptosis of cancerous cells. A magnetic field of 0.1 mTesla to 10 mTesla is generally used for this purpose. Uses pulsed electromagnetic fields only. Whereas in the present invention relates to a magneto-acoustic apparatus that synchronously uses LIPUS and SMF, which are absent in US6926659B1**.**

Reference may be made to US20220001172A1 (Apparatus and method for treating multiple tumors in patients with metastatic disease by electric fields) wherein, a method of using an electrode array to deliver tumor treating electric fields to a patient including the steps of electrode placement, programming, locating, etc. Whereas in the present invention relates to a magneto-acoustic apparatus that synchronously uses LIPUS and SMF.

Reference may be made to US7410469B1 (Apparatus and method for ultrasonically and electromagnetically treating tissue) and EP1180057B1 (Apparatus for ultrasonically and electromagnetically treating tissue) wherein, the disclosure relates to apparatus and method for ultrasonically and electromagnetically treating traumatized tissue or a bone injury. The apparatus includes one ultrasonic transducer assembly along with one electromagnetic coil assembly configured cooperate with a placement module for placement in proximity to the treatment area. The activation of the at least one ultrasonic transducer and the at least one electromagnetic coil may be performed at the same time or at different times for varying periods. Here the use of continuous ultrasound and pulsed electromagnetic is reported and no mention about their parameters and do not use pulsed ultrasound and SMF synchronously as in the present invention of magneto-acoustic apparatus.

***However, no prior patent disclosures are related to present invention on non-invasive magneto-acoustic apparatus that combines LIPUS and SMF to generate in situ electro-mechanical stimuli thereby modulating cellular activities for accelerated cartilage, bone healing, regeneration and inhibiting cancer cells for efficient cancer treatment.***

### OBJECTIVE OF THE INVENTION:

*The main object of the present invention is to provide a non-invasive magneto-acoustic apparatus for healthy tissue healing, regeneration and cancer regression which obviates the drawbacks of the hitherto known prior at as detailed above.*

Another object of the invention is to synchronously uses the low intensity pulsed ultrasound (LIPUS) and static magnetic field (SMF), provide conducive cellular environment and offer accelerated regeneration, repair of bone, cartilage and skin through selectively activate cellular activities such as rapid cell proliferation, differentiation, extra cellular matrix (ECM) production, cell membrane hyperpolarization and upregulating specific genes, specific to cartilage, bone, skin leading to accelerated tissue healing, regeneration, when used appropriately..

Yet another object of the invention is to selectively targeting and inhibiting rapidly proliferating cancer cells such as cancers of breast, skin, bone leading to enhanced cancer regression through cell membrane depolarization, cell cycle arrest and death, over production of reactive oxygen species (ROS), , while leaving the healthy cells unaffected, using magneto-acoustic apparatus appropriately..

Another object is to selectively tailoring the cellular activities by treating the desired tissue, tumor with simultaneously exposure of pulsed ultrasound and magnetic field for sufficient duration each day, for multiple days, using magneto-acoustic apparatus with appropriate parameters.

Another object of using the magneto-acoustic apparatus for treatment is to generate in-situ electro-mechanical stimuli, in the portion of the selected tissue, and modulate several cellular activities.

Still another object of the magneto-acoustic apparatus use, as an adjuvant, is to enhance current cancer treatment efficiency and reduce side effects of anticancer drugs.

Still another object of the present invention is to provide simple magneto-acoustic apparatus, units and sequential steps of its economic use for tissue regeneration and cancer regression.

### SUMMARY OF THE INVENTION

Accordingly, the main aspect of the present invention provides a non-invasive magneto-acoustic apparatus for healthy tissue healing, regeneration and cancer regression which comprises synchronous use of low intensity pulsed ultrasound (LIPUS) and static magnetic field (SMF), as an adjuvant in conjunction with current treatments or standalone, for therapeutically treating degenerated, injured tissues (cartilage, bone, skin) and diseased tissues (breast, skin, bone cancer), to generate in-situ generated electro-mechanical stimuli and modulate several cellular activities, for sufficient duration each day, for multiple days, to provide conducive cellular environment for accelerated regeneration, repair of bone, cartilage, skin due to in-situ electro-mechanical stimuli mediated rapid cell proliferation, differentiation, extra cellular matrix (ECM) production, cell membrane hyperpolarization and upregulation of specific genes, and to selectively target and inhibit rapidly proliferating cells such as cancer (breast, skin, bone) through cell membrane depolarization, cell cycle arrest and death, over production of reactive oxygen species (ROS).

In another aspect of the invention, the present invention discloses a non-invasive magneto-acoustic apparatus combines the application of LIPUS and SMF, their parameters and method of use established in this invention is of great importance due to its non-invasive nature and in-situ generation of electro-mechanical stimuli to activate cellular activities towards desired outcome. The magneto-acoustic treatment with simultaneous application of LIPUS and SMF (using permanent magnets (NdFeB)) for duration up to 45 minutes every day, for several days, provide conducive cellular environment for rapid cell proliferation, differentiation, extra cellular matrix (ECM) production, cell membrane hyperpolarization and upregulating specific genes pertinent to regeneration/repair of bone, cartilage and skin. While the same treatment provides cellular environment that can selectively inhibit rapidly proliferating cells such as cancer (breast, skin and bone) through cell membrane depolarization, cell cycle arrest and death, over production of reactive oxygen species (ROS). Further acceleration of healthy tissue healing/repair (bone, cartilage and skin) or tumour regression (breast, skin and bone) can be achieved through optional use of appropriate concentration of standard osteogenic or chondrogenic or fibroblastic supplements for bone, cartilage and skin, respectively, and appropriate concentration of standard anticancer treatment drugs.

In another aspect of the invention, magneto-acoustic apparatus with unique combination of LIPUS and SMF, with optional supplements, for non-invasive accelerated healing of multiple tissues such as bone, cartilage and skin, and is highly selective towards inhibiting multiple cancers (cancer cells, breast, skin and bone) while leaving normal cells unaffected.

In another aspect of the invention, magneto-acoustic apparatus with broad range of LIPUS parameters and SMF field strengths with or without supplements (osteogenic, chondrogenic and fibroblastic supplement(s) for bone, cartilage and skin healing/ regeneration, respectively; and anticancer drug(s) for treatment of breast, skin, bone cancer) for desired/ patient specific treatment outcome.

The present invention discloses a non-invasive magneto-acoustic apparatus, for tissue regeneration and cancer regression comprises the components of:
(a) at least one pulsed ultrasound unit (2) wherein the pulsed ultrasound unit (2) comprising:
   (i) one or more ultrasound probe (5) for generating pulsed ultrasound intensity; and
   (ii) at least one permanent magnets (6) connected through cable (13) for generating the static magnetic fields;
(b) a power supply unit (1);
(c) at least one control system (3) connected with control system interface (4) for controlling the parameters of the pulsed ultrasound unit (2);
(d) at least one module housing (7) for accommodation of pulsed ultrasound unit (2);
(e) at least one housing fixture (8) for positioning of the pulsed ultrasound probe (5) and permanent magnets (6) at predetermined orientation (9) with respect to each other and region of damaged tissue to be treated.

Other aspect of the present invention provides the housing module (7) further comprising flexible straps (10) with inserts (12) at either end of the housing with knob (11).

Another aspect of the present invention provides the pulsed ultrasound unit (2) having the pulsed ultrasound frequency in the range of 1 to about 3 MHz and ultrasound pulse repeating frequency in the range of 0.5 to about 2 kHz.

In yet another aspect of the present invention provides the ultrasound intensity having the range of 10 to about 100 mW/cm².

In other aspect, the pulsed ultrasound unit (2) is having an ultrasound pulse width in the range of 100 to about 200µs.

In yet other aspect provides the ultrasound probe (5) having a diameter in the range of 10-30 mm

In one of the embodiment of the present invention discloses the permanent magnets (6) having a static magnetic field selected from the range of about 50 to about 500 mT.

In another embodiment of the present invention discloses the positioning of the ultrasound probe (5) and permanent magnets (6) at an angle from about 90 to about 180°.

In yet other embodiment of the present invention discloses the magneto-acoustic apparatus comprising the steps of inserting the ultrasound transducers (5) and permanent magnets (6) inside the module housing (7) and adjusting orientation θ between the axis of ultrasound probes (5) and permanent magnets (6) inside the housing fixture (8).

In yet another embodiment of the present invention discloses the use of magneto-acoustic apparatus in tissue regeneration in cancer treatment.

### DESCRIPTION OF DRAWINGS:

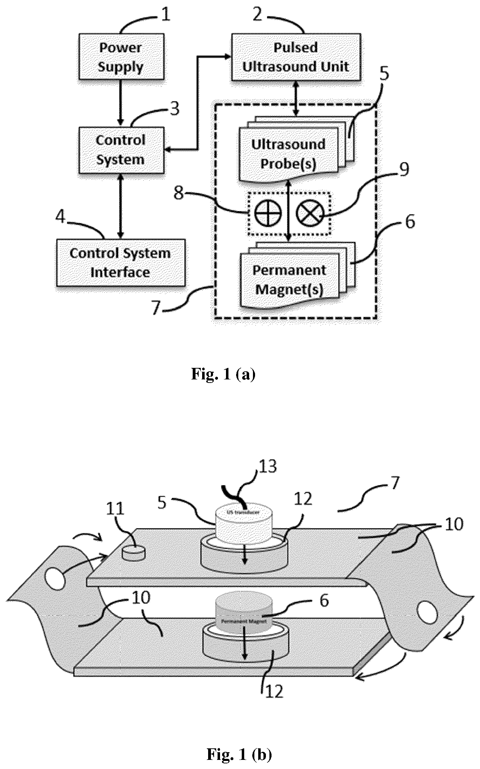

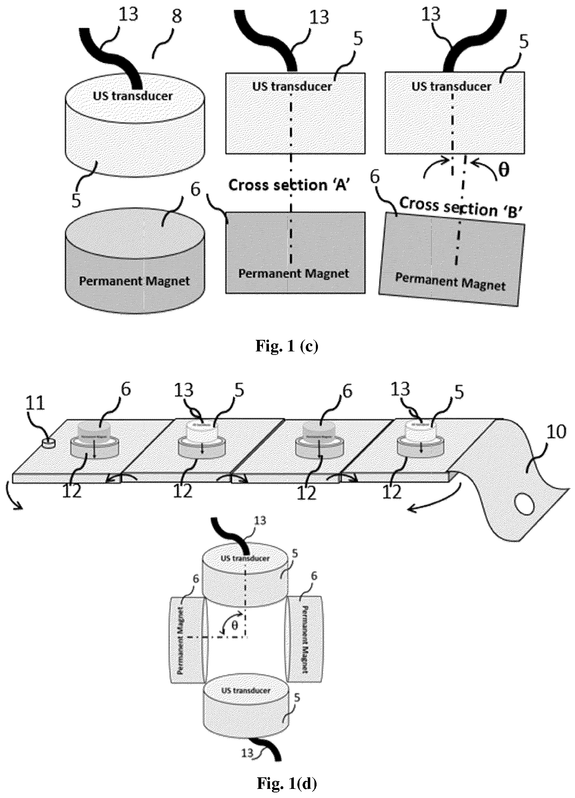

*The present invention is illustrated in* *Figure 1 to Figure 5* *of the drawing(s) accompanying this specification. In the drawings like reference numbers*/*letters indicate corresponding parts in the various figures. The drawings are included to provide better understanding of the invention, illustrate the embodiments of the invention and together with the descriptions serve to explain the importance of the invention.*
**Fig.1****:** (a) Schematic drawing showing the architecture of magneto-acoustic apparatus disclosed in this invention, (b) View of the Module Housing (7) to accommodate ultrasound probe(s) (5) and permanent magnet(s) (6) of magneto-acoustic apparatus, (c) View of the Housing Fixture (8) with arrangement(s)/orientation(s) (9) of ultrasound probe (5) and permanent magnet (6) of magneto-acoustic apparatus, (d) View of the Housing Fixture (8) with arrangement(s)/ orientation(s) (9) of multiple ultrasound probes (5) and permanent magnets (6) of magneto-acoustic apparatus, as per an embodiment herein.

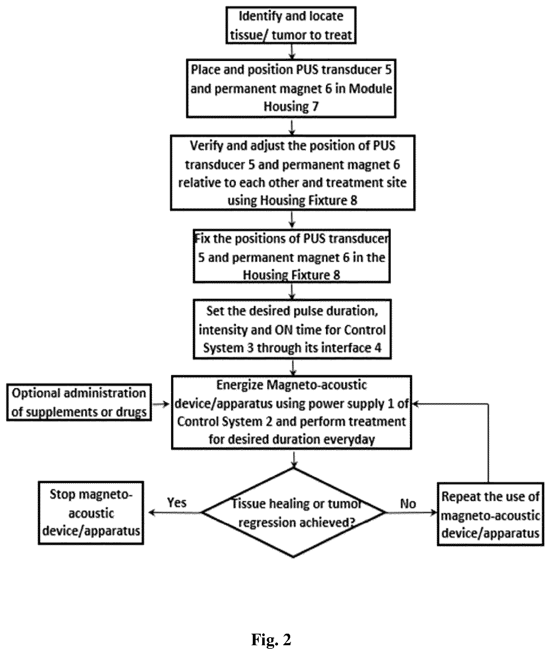

**Fig. 2****:** Flowchart illustrating logical sequence of steps implemented in using magneto- acoustic apparatus to provide in situ generated electro-mechanical stimuli to damaged tissue (bone, cartilage, skin, tumor) to accelerate tissue healing and/ or tumore regression selectively, as per an embodiment herein.

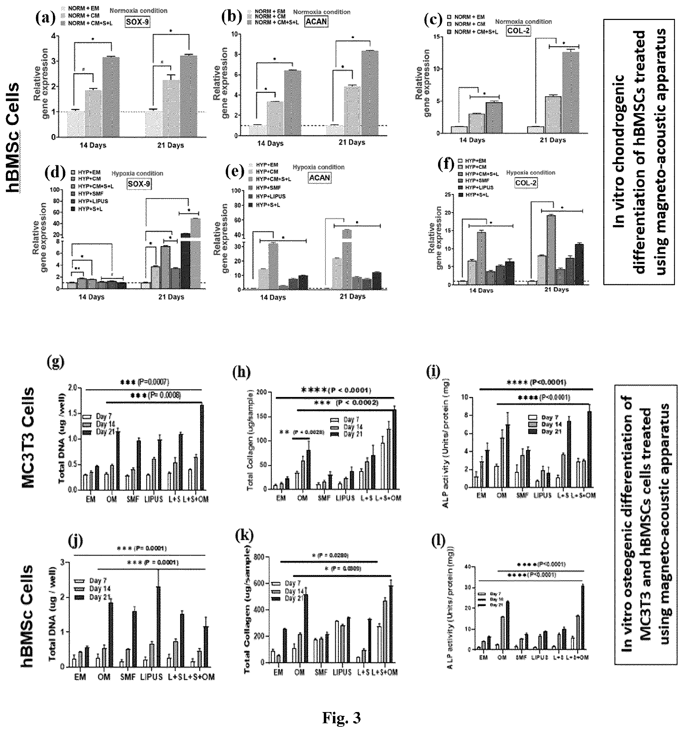

**Fig. 3****:** (a-f) Condrogenic differentiation of hBMSCs. Relative gene expression profiles of cartilage specific genes in normoxia condition (hBMSc) (a) SOX-9 (b) Aggrecan (c) Collagen 2 and the expression of same genes in hypoxia condition (hBMSc) (d) SOX-9 (e) Aggrecan (f) Collagen 2, for the different experimental groups [# P>0.05, * P<0.05, statistical analysis was done by one-way ANOVA analysis and pair comparison was made between Normoxia + EM vs. remaining sample groups and hypoxia + EM vs. remaining sample groups ], as per an embodiment herein. (g-i) Osteogenic differentiation of MC3T3 cells (g) Estimation of total DNA by DNA-Pico green assay (h) Estimation of total collagen normalisation by total protein (i) ALP estimation assay. [EM, OM, SMF @ 200 mT (S), LIPUS @ 30mW/cm² (L), LIPUS @ 30mW/cm² along with SMF @ 200 mT (L+S i.e., magneto-acoustic apparatus) and LIPUS @ 30mW/cm² and SMF @ 200 mT along with Osteogenic supplement (L+S+OM i.e., magneto-acoustic apparatus)]. (j-l) Osteogneic differentiation of hBMSc cells (j) Estimation of total DNA by DNA-Pico green assay, (k) Estimation of total collagen, (1) ALP activity on hBMSc cell line [* P ≤ 0.05, ** P ≤ 0.01, *** P ≤ 0.001, **** P ≤ 0.0001], as per an embodiment herein.

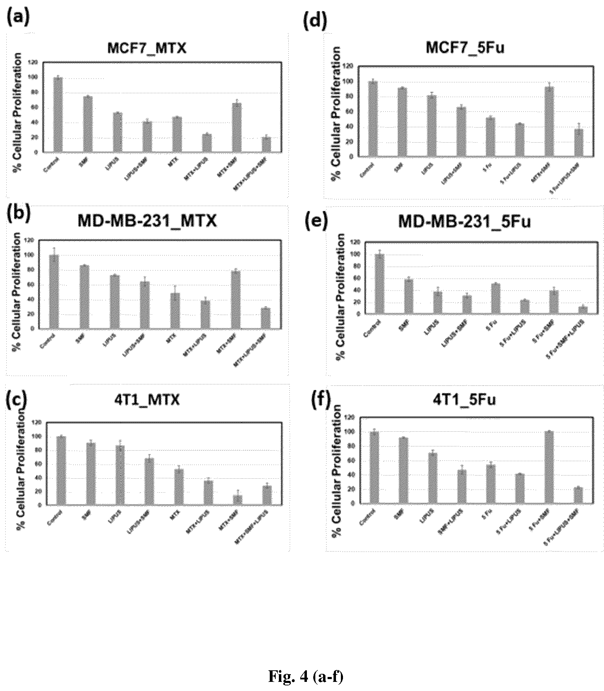

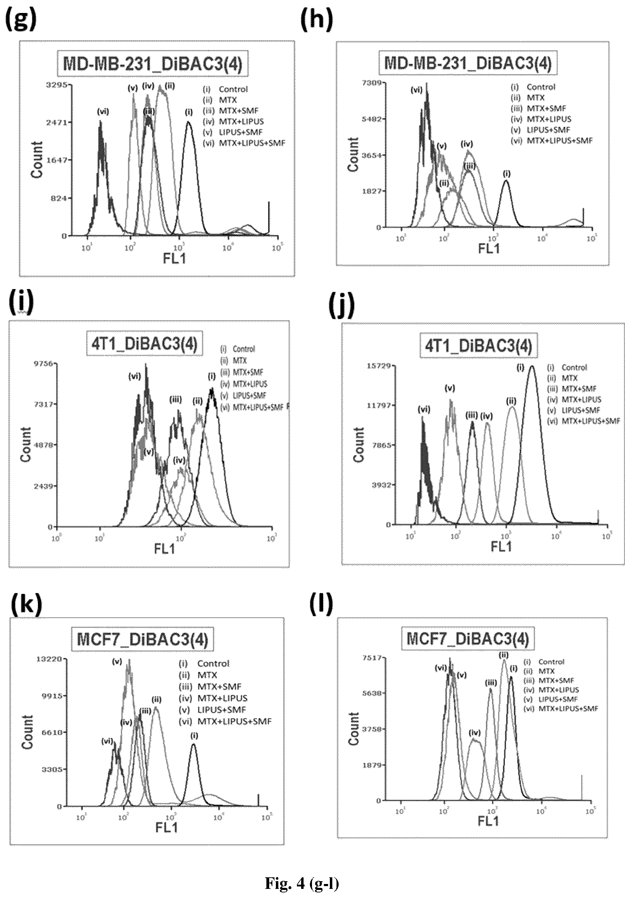

**Fig. 4****:** (a-f) Cellular inhibition of different breast cancer cells, after 72 h of culture, under different treatment conditions with and without the presence of anticancer drugs at IC50 concentration (a,c,e) with MTX and (b,d,f) with 5Fu. (a,b) MCF7 cells, (c,d) MDMB231 cells (e,f) 4T1 cells, (g-l) Changes in the membrane potential determined using voltage-sensitive bis(1,3-dibutylbarbituric acid) trimethineoxonol (DiBAC4(3)) dye (g,i,k) with MTX and (h,j,l) with 5Fu. (g,h) MC7 cells, (i,j) MDMB231 cells (k,l) 4T1 cells, as per an embodiment herein.

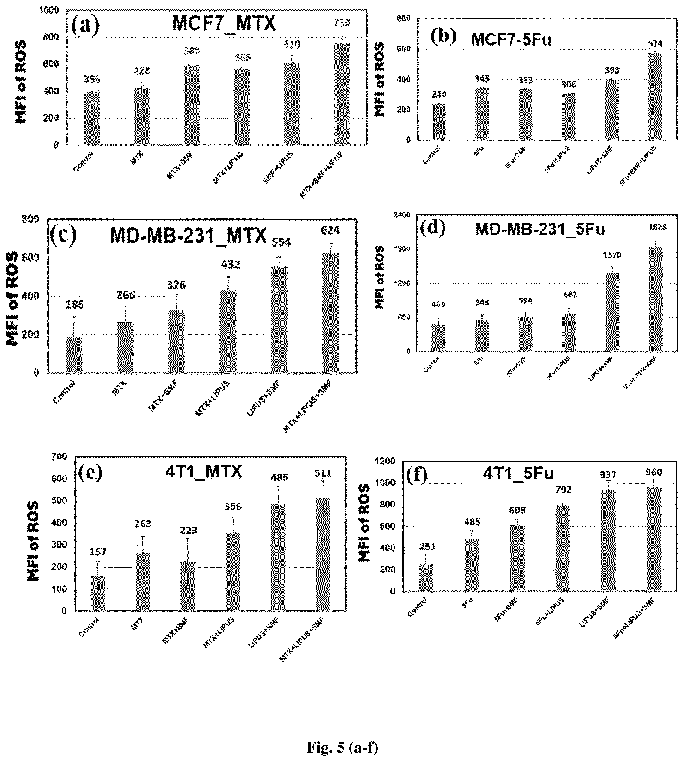

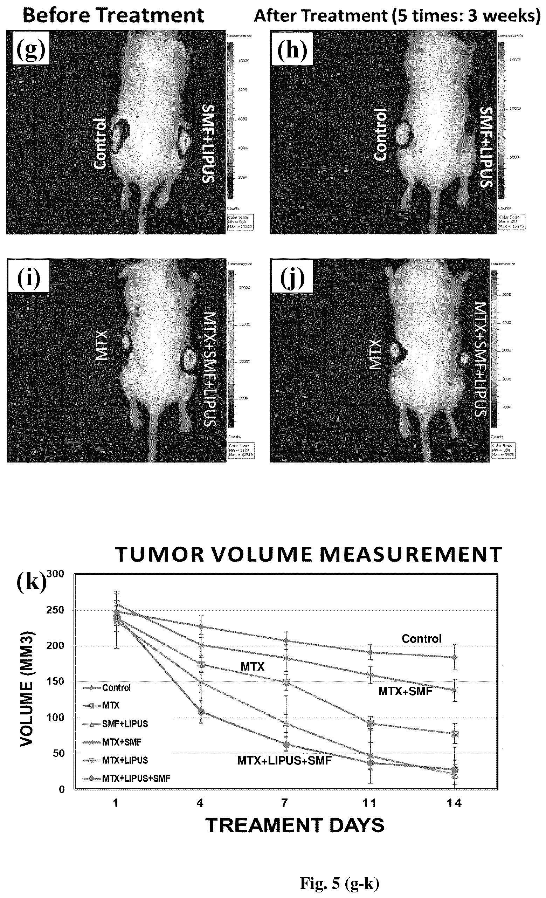

**Fig. 5****:** (a-f) Measured intracellular ROS in terms of mean fluorescence intensity (MFI) (a,c,e) with MTX and (b,d,f) 5Fu. (a,b) MCF7 cells, (c,d) MDMB231 cells (e,f) 4T1 cells, (g-j) Bioluminescence images of sub-cutaneous breast tumor in bulb/c mice (g,i) before treatment (h,j) after treatment. The Groups are Control, MTX, MTX+LIPUS+SMF (magneto-acoustic apparatus) and LIPUS+SMF (magneto-acoustic apparatus). (k) Changes in the tumor volume in different Groups as a function treatment days, as per an embodiment herein.

### DETAILED DESCRIPTION OF THE INVENTION

According to the present invention, non-invasively treating degenerated, injured tissues namely cartilage, bone, skin, with combined exposure of pulsed ultrasound (LIPUS) and static magnetic field (SMF) using magneto-acoustic apparatus can upregulate, activate the cellular activities specific to these tissues, cells due to synergistic influence of in-situ generated electro-mechanical stimuli. The same non-invasive apparatus can selectively downregulate, inhibit cellular activities specific to diseased tissues such as cancers of breast, skin, bone while leaving the healthy, normal cells if any, around the diseased tissues, unaffected. The apparatus can also be used an adjuvant in conjunction with current treatments for healing degenerated/injured tissues or inhibiting, regression of cancer.

The present invention provides magneto-acoustic apparatus comprises a pulsed ultrasound unit with appropriate probe or transducer diameter to generate ultrasound of desired frequency and pulse frequency, control system for pulsed ultrasound unit to energize the unit with desired pulse duration, intensity and active/ON time, permanent magnets with desired field strength and size to generate static magnetic fields, module housing to accommodate ultrasound probe and permanent magnets, housing fixture to position ultrasound probe and permanent magnets at desired orientation with respect to each other and region of tissue.

The present invention as illustrated in **Fig. 1a** shows schematic architecture of magneto-acoustic apparatus disclosed in this invention. The apparatus has one or more pulsed ultrasound (PUS) units (2) connected to control system (3) with interface (4) to control PUS parameters such as pulse duration, intensity and active/ON time. The PUS unit (2) can be energized using a power supply (1). The PUS units have one or more transducers/probes (5) which are inserted in module housing (7). The module housing also comprises provision for inserting permanent magnets (6). The arrangement and orientation (9) of PUS transducers/probes and permanent magnets are arranged in housing fixture (8).

The present invention as illustrated in **Fig. 1b** shows the details module housing 7 to hold PUS transducers (5) and permanent magnets (6). The module housing comprises flexible strap (10) with inserts (12) to hold transducers (5) and permanent magnets (6). The flexible strap (10) may be used to hold the transducers 5 and permanent magnets (6) together and against skin or tissue of interest. The strap (10) is wrapped around the tissue of interest and locked with knob (11). The PUS transducers (5) are connected to PUS unit (2) through cable (13). The present invention as illustrated in **Fig. 1c** provides a view of the Housing Fixture 8 with arrangement(s)/ orientation(s) (9) of ultrasound transducers (5) and permanent magnets (6) of magneto-acoustic apparatus. The housing fixture (8) enable adjusting the orientation θ between the axis of ultrasound probes (5) and permanent magnets (6). The orientation θ can also be referred to as the orientation between the direction of PUS wave propagation and the static magnetic field axis. The orientation θ can range from about 90 to about 180°.

The present invention as illustrated in **Fig. 1d** above shows the arrangement of multiple ultrasound transducers (5) and permanent magnets (6) inside the housing fixture. The flexible strap (10) with multiple ultrasound transducers (5) and permanent magnets (6) housed in respective inserts (12) can be wrapped around the tissue of interest. This housing fixture is used to adjust the orientation θ between the axis of ultrasound probes (5) and permanent magnets (6).

In another embodiment, the use of magneto-acoustic apparatus comprises inserting the ultrasound transducers (5) and permanent magnets (6) inside the module housing (7) and adjusting orientation θ between the axis of ultrasound probes (5) and permanent magnets (6) inside the housing fixture (8). The module housing (7) along with the housing fixture (8) is wrapped with strip (10) around the tissue of interest, touching the skin, and locked against the knob (11). The parameters pulse duration, PUS intensity/power, PUS active/ON time are set for the PUS unit through control system interface (4) provided on control system (3).

In another embodiment, the magneto-acoustic apparatus is energised using power supply (1) and then the stimulating PUS will be propagated to the desired tissue though ultrasound transducers (5) and the static magnetic fields through permanent magnets (6). The synchronous application of PUS and static magnetic field generate in situ electromechanical stimuli within the tissue/ tumor. The scientific mechanism behind in-situ generation of electro-mechanical stimuli in the cells/tissue/tumor due to synchronous PUS and SMF application through disclosed magneto-acoustic apparatus, is that the charged ions of extracellular milieu or tissue experience Lorentz force due to PUS induced acoustic wave oscillation of the tissue/ions and in the presence of SMF. This force separates the ions/charges in the opposite direction thus generate local electrical field proportional to the electrical conductivity of the tissue. The synergy between these in-situ electrical and mechanical stimuli (electro-mechanical stimuli) can modulate several cellular activities of chondrogenic/osteogenic precursor cells including transmembrane signals, stimulate growth factors, and consequently cartilage/bone healing/regeneration and inhibit cancer cells by altering their cell cycle, apoptosis, cell membrane destabilization, elevated ROS production. In another embodiment, the induced electro-mechanical stimuli in the target tissue/tumor may result in the following physiologic effects:
(1) Accelerated hBMSC cell differentiation towards cartilage/ chondrogenesis in vitro through enhanced sGAG, sGAG:DNA ratio, nodule formation in Alcian blue staining, production of total collagen, SOX-9, Aggrecan, and Collagen 2 (Example 2). (2) Accelerated osteoblast precursor cells (MC3T3)/ hBMSCs towards osteogenesis through enhanced cell viability, proliferation and early stage osteogenesis (Example 3). (3) Selective and rapid inhibition of breast cancer cells (MCF7, MDMB231 and 4T1) in vitro, while leaving healthy cells unaffected, through enhanced model drugs intake as a result of depolarization of cell membranes leading to mitotic cell arrest followed by cell death (Example 4) and under in vivo conditions significant breast cancer tumor regression both in the presence and absence of model drugs (Example 5).

In another embodiment, the duration of use of this magneto-acoustic apparatus, for tissue healing/regeneration or tumor regression, can be up to 45 minutes each day and the number of days for treatment can be determined depending on the clinical outcome, can vary from days to weeks to months. The disclosed magneto-acoustic apparatus can be used as standalone or in conjunction with (simultaneously or before or after) other methods currently in use for tissue repair/healing/ regeneration or cancer treatment.

In another embodiment, the use of magneto-acoustic apparatus comprising essential steps of (Example 1): a) Placing pulsed ultrasound (PUS) probe/ transducer on the region of tissue/ tumor to expose it to PUS. b) Placing permanent magnet on the region of tissue/ tumor to expose it to static magnetic field (SMF) aligned at desired orientation with respect to pulsed ultrasound and tissue/target site. c) Continued use of magneto-acoustic apparatus to expose the tissue/ tumor, to PUS+SMF, every day for desired duration, for several days or weeks or months, to accelerate tissue healing and/ or tissue regression, selectively. d) Use of magneto-acoustic apparatus before or after other methods currently in use for tissue repair/healing/ regeneration or cancer treatment. e) Optional use of osteogenic, chondrogenic and fibroblastic supplement(s) for bone, cartilage and skin healing/ regeneration, respectively, in conjunction with magneto-acoustic apparatus. f) Optional use of anticancer drug(s) for cancer treatment of breast, skin, bone, in conjunction with magneto-acoustic apparatus.

In one of the embodiment of present invention, human bone marrow derived mesenchymal stem cells (hBMSCs) were used in *in vitro* cell culture model (Example 2) and exposed them to magneto-acoustic apparatus (LIPUS+SMF) (up to 100 mW/cm², up to 500 mT, up to 45 min/day) for different culture durations under normoxia (37 °C, 5% CO₂) and hypoxia. These experiments demonstrated positive influence of the magneto-acoustic apparatus use on accelerated hBMSC cell differentiation towards cartilage in vitro through enhanced sGAG, sGAG:DNA ratio, nodule formation in Alcian blue staining, total collagen and hypoxia condition further enhanced the cell differentiation towards cartilage. The RT-qPCR results demonstrated several fold increases in the cartilage specific genes namely SOX-9, Aggrecan, and Collagen 2. Another embodiment consists of treating the mouse osteoblast precursor cells (MC3T3) and hBMSCs using magneto-acoustic apparatus (Example 3) which found to significantly enhance the cell viability, proliferation and early-stage osteogenesis. The use of this apparatus tends to hyperpolarize the healthy cells, alter intracellular ROS production and enhanced bone mineralization through Alizarin red S staining. The PUS intensity below 100 mW/cm² cannot create micro-streaming or cavitation, but pressure and mechanical vibrations can alter cell membrane permeability towards Na+/Ca+ ions leading to hyperpolarization and thus improves cell proliferation followed by mineralization. The in-situ generated electrical stimuli, due to the use of magneto-acoustic apparatus, triggers further activation of cellular activities. The use of disclosed magneto-acoustic apparatus has potential to accelerate chondrogenesis/osteogenesis by tailoring diverse cellular functions.

Another embodiment discloses that invented magneto-acoustic apparatus use can also rapidly inhibit breast cancer cells (MCF7, MDMB231 and 4T1) in vitro (Example 4). The rapid inhibition is obtained through enhanced model drugs (methotrexate and 5 fluorouracil) intake (about 99% of administered drug) by these cells as a result of depolarization of cell membranes leading to mitotic cell arrest followed by cell death. The cellular inhibition up to 80% can be achieved within 72 hours (3 days) of culture when used with the invented magneto-acoustic apparatus for 30 min/day.

Another embodiment, the use of this apparatus on female mice with subcutaneous breast cancer (Example 5) reduced the tumor volume from 250 mm³ to ~ 25 mm³ in 14 days (twice a week use for 30 min./day at 96 h interval) compared to control (drug alone ~ 180 mm³). The apparatus use without drug reduced the tumor to similar extent at 14 days, but slowly up to 7 days. The present invention comprising the use of LIPUS in the presence of SMF through magneto-acoustic apparatus, is different from high intensity focused ultrasound (HIFU), which depends on hyperthermia to kill cancer cells. The intensity of HIFU typically range between 100 and 10000 W/cm² and can generate heat up to 80°C in the exposed tissue leading to potential damage to healthy tissue/cells. Present apparatus with PUS (up to 100 mW/cm²) is highly selective to cancer cells and inhibit cells non-thermally. The inhibition of healthy cells with the use of present magneto-acoustic apparatus is very minimal (less than 1% without anticancer drug supplements and less than 10% in the presence of anticancer drug supplements). The temperature variation during the use of this apparatus can be in the range about 1 to 3 °C.

In another embodiment, in vitro cartilage, bone regeneration properties and in vitro and in vivo cancer cell inhibition and tumor regression observed using disclosed magneto-acoustic apparatus are provided in Examples 2, 3, 4 and 5. It is understood that within the scope and embodiments of the present invention different modifications can be made - type of ultrasound and its parameters, method of generating static magnetic field, type of magnetic field (pulsed), treatment duration and frequency, etc. Therefore, the above detailed description presents selected examples of the invention and is not limiting the scope of the present invention claims.

### EXAMPLES

### The following examples are given by way of illustration only and therefore should not be construed to limit the scope of the present invention

### Example 1

The use of magneto-acoustic apparatus, combining low intensity pulsed ultrasound (LIPUS) and static magnetic field (SMF), to generate in situ generated electro-mechanical stimuli in the damaged tissue (bone/ cartilage/ skin/ tumor) for accelerated tissue healing and/ or tumor regression selectively, comprises following sequential steps of (Fig. 2) using elements shown in **Fig. 1****:** a) Identifying the tissue/ tumor to be treated and its location using appropriate imaging technique. b) Placing and positioning the pulsed ultrasound (PUS) probe/ transducer so that the pulsed ultrasound focus point is directed at the identified tissue/ tumor. c) Placing and positioning the permanent magnet(s) of appropriate strength so that the magnetic field covers the identified tissue/ tumor that is being focused by PUS. d) Verifying and adjusting the location of permanent magnet(s) with desired field strength or combination thereof in relation to the treatment site and tissue/ tumor is covered with magnetic field by selecting/ using appropriate diameter/ size of the permanent magnets(s). e) Verifying and adjusting the PUS probe/ transducer location/ position in relation to treatment site and tissue/ tumor is covered with PUS beam. f) Fixing the positions of PUS probe/ transducer and permanent magnets relative to each other (90° to 180°) and tissue/ tumor with appropriate frame/ locking apparatus to avoid movement of LIPUS probe/ transducer and permanent magnet(s). g) Energizing the PUS unit, using PUS control system, at desired intensity/power, pulse duration, active duration/time or combination thereof in the presence of desired magnetic field strength provided by permanent magnet(s) placed as per Step d. h) Continuing use of energised magneto-acoustic apparatus, exposing the treatment site (tissue/ tumor), for desired duration each day. i) Use of magneto-acoustic apparatus before or after other methods currently in use for tissue repair/healing/ regeneration or cancer treatment. j) Optional use of osteogenic, chondrogenic and fibroblastic supplement(s) for bone, cartilage and skin healing/ regeneration, respectively, in conjunction with magneto-acoustic apparatus. k) Optional use of anticancer drug(s) for cancer treatment of breast, skin, bone, in conjunction with magneto-acoustic apparatus.

### Example 2

### In vitro assessment of chondrogenic differentiation of hBMSCs treated using magneto-acoustic apparatus leading to cartilage regeneration/healing

hBMSCs were thawed and expanded on HiMesoXL^{™} Mesenchymal stem cell expansion media (HiMedia, India) with 10% of FBS and 1% of Antimycotic/Antibiotic solution (HiMedia, India) and incubated at 37°C and 5% CO₂. Six groups were considered for this study as shown in Table 1 below. Each group was seeded at a density of 65,000 cells/well in 6 well plate and the cultures were maintained up to 21 days under normoxia (37°C, 5% CO₂, 21.7% O₂) and hypoxia (37°C, 5% CO₂ and 3% O₂) and the medium was fully renewed every 3-4 days. In case of Group - 2 and Group - 6, sufficient amount of HiChondroXL^{™} (HiMedia, India) Chondrogenic Differentiation Supplement was added to the base media. The treatment (SMF, LIPUS and SMF+LIPUS i.e., magneto-acoustic apparatus) groups were exposed to the physical stimuli for 15 min./day during the entire culture duration. Permanent magnets (NdFeB) were used for static magnetic field (SMF) with a field strength of 200 mT. Similarly, the low intensity pulsed ultrasound (LIPUS, 1.5 MHz) with an intensity of 30 mW/cm² (200 µsec pulse width, 1KHz pulse repletion frequency) was used for the treatments. After the specified incubation period, several assays were performed following standard protocols specified by the manufacturer of the assay kits.

**Table 1: Experimental groups for in vitro magneto-acoustic (LIPUS+SMF) apparatus use under normoxia (NORM) and hypoxia (HYP) conditions. EM: Normal cell culture expansion media; CM: Normal cell culture expansion media supplemented with Chondrogenic media.**

| Group | Cells and media | Treatment |
|---|---|---|
| 1 | hBMSC + EM (negative control) | None |
| 2 | hBMSC + CM (positive control) | None |
| 3 | hBMSC + EM + SMF | SMF only (200 mT) 15 min./alternate day |
| 4 | hBMSC + EM + LIPUS | LIPUS only (30 mW/cm²) 15 min./alternate day |
| 5 | hBMSC + EM + S + L | Magneto-acoustic apparatus: SMF (200 mT) + LIPUS (30 mW/cm²) 15 min./alternate day |
| 6 | hBMSC + CM + S + L | Magneto-acoustic apparatus: SMF (200 mT) + LIPUS (30 mW/cm²) 15 min./alternate day |

The hBMSCs cultured and treated under different conditions were analyzed using important cartilage specific gene expression including SOX9, Aggrecan (ACAN) and Collagen II (COL-2). Under normoxia conditions (Fig. 3a) the relative SOX-9 gene expression increased significantly with treatment (NORM+CM+S+L) compared to other groups, at all culture durations. The LIPUS+SMF treatment increased the SOX-9 gene relative expression to about 3 compared to positive control group (NORM+CM) which had relative expression about 2. Similarly, Aggrecan (ACAN), another cartilage specific gene, also exhibited significantly higher expression, Fig. 3b, compared to positive control group (NORM+CM+S+L). Moreover, the increase in ACAN gene expression is almost twice that of SOX-9 under identical treatment conditions. Further increase in the expression was recorded with magneto-acoustic apparatus treatment for Collagen II (COL-2) gene at 21 days (Fig. 3c). This is attributable to the active role of SOX-9 in controlling the other genes specific to chondrogenesis.

The set of experiments performed under hypoxia condition showed superior expression of all cartilage specific genes (SOX-9, ACAN and COL-2), Fig. 3d-f. Due to avascular nature of the cartilage the oxygen from the synovial fluid can reach the cartilage through diffusion and therefore the concentration of oxygen will be between 9% and 2%. As a result, the hypoxic conditions used in the present experiments (3% O₂) simulate natural chondrogenesis conditions leading to significant increase in the cartilage specific genes. The hypoxia condition also found to decrease the difference between HYP+CM+S+L (positive control) and HYP+S+L in the expression of SOX-9 (Fig. 3d), which control overall chondrogensis individually and through activating other genes such as ACAN and COL-2. Overall superior chondrogensis can be achieved with disclosed magneto-acoustic apparatus both in normoxia and hypoxia with and without chondrogenic supplements compared to other groups.

### Example 3

### In vitro assessment of osteogenic differentiation of MC3T3 and hBMSCs cells treated using magneto-acoustic apparatus leading to bone regeneration/ healing

MC3T3 mouse pre osteoblast cells were thawed and expanded on alpha minimum essential medium (gibco) with 10% of FBS and 1% of Antimycotic/Antibiotic solution (HiMedia, India) and incubated at 37°C and 5% CO₂. The hBMSC cell line was expanded on HiMesoXL^{™} Mesenchymal stem cell expansion media with an equal quantity of FBS and Antimycotic/Antibiotic solution in the same condition. Total Six groups were considered for the study as shown in Table 2. Each group was seeded at a density of 65,000 cells/well in 6 well plate and the cultures were maintained up to 21 days under normoxia (37°C, 5% CO₂, 20.7% O₂) and the medium was changed in every 3-4 days. For group OM and (L+S+OM i.e., magneto-acoustic apparatus), sufficient amount of HiOsteoXLTM (HiMedia, India), Osteogenic Differentiation Supplement was added to the normal α- MEM media prepared earlier. The treatment (SMF, LIPUS and L+S i.e., magneto-acoustic apparatus, L+S+OM i.e., magneto-acoustic apparatus) groups were exposed to the physical stimuli for 15 min/ alternative day during the entire culture duration starting from the next day where 0 is the cell seeding day. SMF strength and LIPUS parameters were identical to those used for Example 2. After the specified incubation period several assays were performed following standard protocols specified by the manufacturer of the assay kits.

**Table 2: Experimental groups for in vitro assessment of osteogenic differentiation of MC3T3 and hBMSCs cells exposed to different magneto-acoustic apparatus treatment conditions along with positive and negative controls. (EM: Normal cell culture expansion media; OM: α -MEM media with osteogenic supplement)**

| **Group** | **Cells and media** | **Treatment** |
|---|---|---|
| 1 | MC3T3 or hBMSCs + EM (EM) [Negative control] | None |
| 2 | MC3T3 or hBMSCs + OM (OM) [Positive control] | None |
| 3 | MC3T3 or hBMSCs + EM + SMF (SMF) | SMF only (200 mT) 15 min / alternate day |
| 4 | MC3T3 or hBMSCs + EM + LIPUS (LIPUS) | LIPUS only (30 mw/cm²) 15 min/ alternate day |
| 5 | MC3T3 or hBMSCs + EM + S + L (L+S) | Magneto-acoustic apparatus: SMF (200 mT) + LIPUS (30 mW/cm²) 15 min/ alternate day |
| 6 | MC3T3 or hBMSCs + OM + S + L (L+S+OM) | Magneto-acoustic apparatus: SMF (200 mT) + LIPUS (30 mW/cm²) 15 alternate day |

(i) In vitro assessment of osteogenic differentiation of MC3T3 exposed to different treatment conditions along with positive and negative controls: Total DNA estimation by Pico Green dsDNA assay results (Fig. 3g) that clearly demonstrate the treatment conditions (LIPUS, SMF, L+S i.e., magneto-acoustic apparatus, and L+S+OM i.e., magneto-acoustic apparatus) can significantly enhance the cell proliferation when compared with negative control group. Overall, all the sample groups showed increase in cell numbers with increasing culture period from day 7 to day 21. Interestingly, when compared with no treatment group with normal expansion medium (EM) at Day 21 time point it has been found that almost 2.29-fold, 2.08-fold, and 2.05-fold increase in DNA content for L+S i.e., magneto-acoustic apparatus, L, and S treatment group, respectively. Moreover, the positive sample group with osteogenic supplemented medium but without treatment (OM) showed an increase of 2.39-fold when compared with normal expansion medium sample group (EM). However, the highest increase of 3.45-fold in DNA content can be noticed for L+S+OM sample group (magneto-acoustic apparatus) than only EM group indicating that the magneto-acoustic apparatuswas found to be most effective in presence of osteogenic supplemented medium (OM) for increasing the growth of cells. Furthermore, the total collagen content was found similar in (Fig 3h) amongst treatment groups (i.e., S, L and L+S i.e., magneto-acoustic apparatus) and osteogenic supplemented group (OM i.e. positive control). However, the total collagen content was found to be maximum (80% of total protein) for L+S+OM sample group (i.e., magneto-acoustic apparatus) at Day 21 with almost 2.91-fold and 1.38-fold increase in total collagen content when compared with EM and OM groups, respectively. Furthermore, the expression of alkaline phosphatase increased (Fig. 3i), with respect to the negative control group (EM), in all treatment groups (LIPUS, SMF, L+S i.e., magneto-acoustic apparatus, and L+S+OM i.e., magneto-acoustic apparatus). It's interesting to note that by Day 21, the ALP (normalized by total protein) content of the L+S (magneto-acoustic apparatus), L, and S treatment groups was found to be about 1.81-fold, 1.01-fold, and 0.4-fold higher, respectively, than that of the no treatment group when using normal expansion medium (EM). Additionally, compared to the normal expansion medium sample group (EM), the positive sample group with osteogenic supplemented medium but no therapy (OM) showed a 1.7-fold increase.
(ii) In vitro assessment of osteogenic differentiation of hBMSCs exposed to different treatment conditions along with positive and negative controls: Total DNA estimation by Pico Green dsDNA assay results (Fig. **3j**) clearly demonstrated that the treatment conditions (LIPUS, SMF, L+S i.e., magneto-acoustic apparatus, and L+S+OM i.e., magneto-acoustic apparatus) can significantly enhance cell proliferation when compared with the negative control group. At Day 21, the total DNA content of the L+S (magneto-acoustic apparatus), L, and S treatment groups increased by about 2.69 folds, 4.1 folds, and 2.84 folds, respectively, in comparison to the control group that received no treatment with normal expansion medium (EM). Furthermore, the positive sample group utilising osteogenic supplemented medium without treatment (OM) demonstrated a 3.29-fold increase in comparison to the normal expansion medium sample group (EM). Furthermore, the total collagen content was found to be similar (Fig. 3k) among the treatment groups (i.e., S, L, and L+S i.e., magneto-acoustic apparatus) and the osteogenic supplemented group (OM, i.e., positive control). On Day 21, the total collagen content of L+S+OM (magneto-acoustic apparatus) reached its maximum (80% of total protein), increasing by about 1.36 and 0.77 folds, respectively, in comparison to the EM and OM groups. In summary, our findings clearly suggest that the synergistic effects of magneto-acoustic apparatus in the presence of osteogenic supplements is able to enhance the collagen-like extracellular matrix (ECM) as well as increase cell proliferation and mineralization. ALP is an early marker of osteogenic differentiation, which is associated with the mineralization of bone tissues, and is an indicator for confirming the differentiation of pre-osteoblast cells. Fig. 31 show that by Day 21, the ALP (normalized by total protein) content of the L+S (magneto-acoustic apparatus), L, and S treatment groups was about 1.4-fold, 1.2-fold, and 1.4-fold higher, respectively, than that of the no treatment group when using normal expansion medium (EM). Additionally, compared to the normal expansion medium sample group (EM), the positive sample group with osteogenic supplemented medium but no therapy (OM) showed a 1.3-fold increase. However, throughout the duration of the treatment, ALP expression increased in all experimental groups, along with positive and negative controls. The study indicates that mineralized cells can induce the differentiation of hBMSCs into osteoblasts i.e., osteogenesis.

### Example 4

### In vitro assessment of breast cancer cell (MCF7, MDMB231 and 4T1) inhibition under the influence of magneto-acoustic apparatus, with and without anticancer drugs

Breast Cancer cells (MCF7, MDMB231, 4T1) and hBMSCs were obtained from ATCC and were routinely cultured in DMEM (Dulbecco's Modified Eagle Medium, Invitrogen, Carlsbad, USA) supplemented with 10% heat-inactivated fetal bovine serum,1 U ml-1 penicillin G and 1 mg ml-1 streptomycin, at 37°C in a humidified atmosphere of 5% CO₂ in an incubator in T75 flask. Eight groups were considered for this study as shown in Table 3 below. Initial cell viability assay was performed on all groups and further detailed assay were carried out on selected groups for effective comparison. The groups with anticancer drugs (MTX: Methotrexate and 5Fu: 5 Fluorouracil) contain experimentally determined IC50 (half maximal inhibitor concentration) of drug in the culture media. Each group was seeded at appropriate density (10,000 cells/well in 6 well plate for MTT assay and 1×10⁶ cells for FACS based assay) and the cultures were maintained up to 3 days under normoxia (37°C, 5% CO₂). The treatment (SMF, LIPUS and SMF+LIPUS i.e., magneto-acoustic apparatus) groups were exposed to the physical stimuli for 30 min./day during the entire culture duration. Permanent magnets (NdFeB) were used for static magnetic field (SMF) with a field strength of 150 mT and LIPUS parameters were identical to those used in Examples 2 and 3. After the specified incubation period (24, 48 and 72 h) different assays were performed following standard protocols specified by the manufacturer of the assay kits.

**Table 3: Experimental groups for in vitro use of magneto-acoustic apparatus for breast cancer cellular inhibition.**

| **Group** | **Details** | **Treatment** |
|---|---|---|
| 1 | Cells only (control) | None |
| 2 | Cells + SMF | SMF only (150 mT) 30 min./day |
| 3 | Cells + LIPUS | LIPUS only (30 mW/cm²) 30 min./day |
| 4 | Cells + LIPUS + SMF | Magneto-acoustic apparatus: SMF (150 mT) + LIPUS (30 mW/cm²) 30 min./day |
| 5 | Cells + Drug (MTX or 5Fu) | None |
| 6 | Cells + Drug + SMF | SMF only (150 mT) 30 min./day |
| 7 | Cells + Drug + LIPUS | LIPUS only (30 mw/cm²) 30 min./day |
| 8 | Cells + Drug + LIPUS + SMF | Magneto-acoustic apparatus: SMF (150 mT) + LIPUS (30 mW/cm²) 30 min./day |

The results of cancer cell inhibition, under different treatment conditions, Fig. 4(a-f), clearly show significantly high cellular inhibition with LIPUS+SMF (magneto-acoustic apparatus) treatment either with or without anticancer drugs after 72 h of culture. Different cell types responded differently to the treatment and resulted in different inhibition effectiveness. Significant amount of cell inhibition (30% to 70%) can be achieved with magneto-acoustic apparatus treatment (Group 4) alone in the absence of anticancer drugs for all cancer cell types. This demonstrate that the invented magneto-acoustic apparatus can be used as standalone treatment for cancer cellular inhibition. The cancer cellular inhibition is further improved, in the range of 65% and 90%, in the presence of anticancer drugs. In all cases the treatment time was 30 min./day and culture duration was 72 h.

The changes in the cell membrane potential measured using voltage-sensitive bis(1,3-dibutylbarbituric acid)trimethineoxonol (DiBAC4(3)) dye and flow cytometry, Fig. 4(g-l), show peak shifting towards left indicating depolarization of cell membrane, compared to control, in all groups. Irrespective of type of cancer cells the disclosed magneto-acoustic apparatus (LIPUS+SMF) depolarized the cell membranes to maximum extent compared to the groups without this treatment. The apparatus is very effective in cell membrane destabilization, through depolarization, both in the presence and absence of anticancer drugs, but marginally better depolarization can be seen with addition of drugs along with magneto-acoustic apparatus treatment. These changes in the membrane potential of cancer cells, depolarization, enable more cross flow of ions and internalization of drugs leading to accelerated cellular death through mitotic arrest.

The production of reactive oxygen species (ROS), measured immediately after the treatment, in terms of mean fluorescence intensity (MFI), Fig. 5(a-f), in the cells significantly increased with magneto-acoustic apparatus treatment. The MFI of control groups varied in the range of 157 and 469, which significantly increased to 511 and 1829 with LIPUS+SMF treatment in the presence of drugs. The influence of MTX and 5Fu drugs appears to be different on ROS depending on the cell type. The 5Fu is relatively more effective in ROS generation in the MDMD231 and 4T1 cells compared to MTX. Although less, the treatment is effective in generating oxidative stress on the cancer cells in the absence of drugs i.e., the ROS in terms of MFI elevated to a range of 398-1370 compared to control group (MFI 157-469). This is very important in terms of reducing or eliminating the side effects of standard anticancer drugs. Further evidence in terms of improved drug intake by the cells, due to cell membrane destabilization, leading to observed enhancement in the cancer cell inhibition is collected through quantification of model drug MTX in the cell lysate using HPLC. The experimental results using MCF7 cells in Group 5 (Cells+MTX) showed MTX concentration of 0 ppm, which increased to about 4 ppm in Group 8 (Cells + MTX + LIPUS + SMF i.e., magneto-acoustic apparatus). While the MDMD231 cells showed further increase in the MTX internalization by the cells, from 0 ppm for Group 5 and 12 ppm for Group 8. This suggests clear improvement in the drug efficiency through enhancement in the cell uptake of the drug leading to more cell inhibition.

### Example 5

### In vivo assessment of breast cancer tumor (4T1) regression under the influence of magneto-acoustic apparatus treatment, with and without anticancer drugs

In vivo experiments were performed using syngeneic mouse model where 6-week-old blub/c female mice having subcutaneous breast cancer after treating with MTX by I.P with a dose of 6.25mg/Kg (½ of Maximum tolerant dose) body weight. The treatment (LIPUS+SMF i.e., magneto-acoustic apparatus, 30 min./day) was given twice a week after 30 min of MTX administration. The tumor volume and body weight were measured along with qualitative analysis of bioluminescence by luciferase assay. The drug was administered once a week and the magneto-acoustic apparatus treatment was given twice a week with 96 h interval. The drug internalization inside the tumor was assessed using HPLC.

The internalization of MTX drug inside the tumor is very effective compared to the cells examined under in vitro conditions. The concentration of MTX inside the tumor increased from 0.506 ppm to about 15.605 ppm when drug was administered along with LIPUS+SMF treatment. This is almost 31 times more internalization of drug by the tumor due to synergistic influence of magneto-acoustic apparatus. The improvement in the drug internalization results in effective tumor regression in LIPUS+SMF treated Groups compared to the Groups without the use of magneto-acoustic apparatus.

Bioluminescence images of breast tumor in bulb/c mice showed qualitative reduction in the tumor size after treatment (Fig. 5(h,j)) compared to those recorded before treatment (Fig. 5(g,i)). The treatment (twice a week treatment at 96h interval) demonstrated to reduce the tumor volume from 250 mm³ to ~ 25 mm³ in 14 days, Fig. 5k, compared to control (MTX alone ~ 180 mm³). The treatment without MTX drug reduced the tumor to similar extent at 14 days, but slowly up to 7 days, Fig. 5k. The results demonstrate that the invented magneto-acoustic apparatus (LIPUS+SMF) is very effective in tumor regression with and without anticancer drugs. The treatment without the use of anticancer drugs can eliminate the side effects of these drugs. Alternatively, the enhanced drug internalization by the tumor due to use of magneto-acoustic apparatus significantly improve the cancer treatment efficiency and the concentration of anticancer drugs can also be reduced to reduce their side effects.

### ADVANTAGES OF THE INVENTION

i. The disclosed magneto-acoustic apparatus comprising simultaneous use of LIPUS and SMF will enable acceleration of bone/cartilage/skin tissue healing/ regeneration non-invasively and can selectively inhibit rapidly proliferating cells pertinent to cancer such as breast, skin and bone.
ii. Non-thermal mechanisms of cancer cell inhibition with magneto-acoustic apparatus is can address thermoresistance of cancer cells due to repeated use of hyperthermia dependent cancer treatments (HIFU, TTFs and PEMFs).
iii. Potential hayline cartilage formation with magneto-acoustic apparatus can eliminate mechanical and tribologically weak fibrous cartilage with current treatment approaches.
iv. Rapid and early integration of artificial implants with host bone tissue due to enhanced osteoinduction leading to new bone formation with magneto-acoustic apparatus.
v. The magneto-acoustic apparatus when used in conjunction with current cancer treatments drugs can reduce the side effects of drugs due to improved treatment/drug efficiency and no side effects when used without drugs.
vi. Magneto-acoustic apparatus with tunable treatment parameters tailored depending on tissue type, defect type, size and tumour type/size enable achieving desired clinical outcome.
vii. Magneto-acoustic apparatus can reduce overall treatment time and costs due to accelerated tissue repair and tumor regression.
viii. The described magneto-acoustic apparatus and method of its use is commercially viable, simple and economic regenerative therapy for bone/skin/cartilage regeneration and also to treat breast/skin/bone cancer.
ix. The magneto-acoustic apparatus is portable and enable on-site treatment by patients and no hospital visits are required.

## Claims

1. A non-invasive magneto-acoustic apparatus for healthy tissue regeneration and cancer regression comprises the components of:
(a) at least one pulsed ultrasound unit (2) wherein the pulsed ultrasound unit (2) comprising
(i) one or more ultrasound probe (5) for generating pulsed ultrasound intensity connected through cable (13); and
(ii) at least one permanent magnets (6) for generating the static magnetic fields;
(b) a power supply unit (1);
(c) at least one control system (3) connected with control system interface (4) for controlling the parameters of the pulsed ultrasound unit (2);
(d) at least one module housing (7) for accommodation of pulsed ultrasound ultrasound probe (5) and permanent magnets (6);
(e) at least one housing fixture (8) for positioning of the pulsed ultrasound probe (5) and permanent magnets (6) at predetermined orientation (9) with respect to each other and region of tissue to be treated.

2. The non-invasive magneto-acoustic apparatus as claimed in claim 1, wherein the housing module (7) further comprising flexible straps (10) with inserts (12) at either end of the housing with knob (11).

3. The non-invasive magneto-acoustic apparatus as claimed in claim 1, wherein the pulsed ultrasound unit (2) is having the pulsed ultrasound frequency in the range of 1 to about 3 MHz and ultrasound pulse repeating frequency in the range of 0.5 to about 2 kHz.

4. The non-invasive magneto-acoustic apparatus as claimed in claim 1, wherein, the ultrasound intensity is having range of 10 to about 100 mW/cm².

5. The non-invasive magneto-acoustic apparatus as claimed in claim 1, wherein, the pulsed ultrasound unit (2) is having a ultrasound pulse width in the range of 100 to about 200µs.

6. The non- invasive magneto-acoustic apparatus as claimed in claim 1 wherein, the ultrasound probe (5) is having a diameter in the range of 10 to about 30 mm.

7. The non-invasive magneto-acoustic apparatus as claimed in claim 1, wherein, the permanent magnets (6) is having a static magnetic field selected from the range of about 50 to about 500 mT.

8. The non-invasive magneto-acoustic apparatus as claimed in claim 1, wherein, the positioning of the ultrasound probe (5) and permanent magnets (6) at an angle from about 90 to about 180°.

9. The non-invasive magneto-acoustic apparatus as claimed in claim 1, wherein the magneto-acoustic apparatus comprising the steps of inserting the ultrasound transducers (5) and permanent magnets (6) inside the module housing (7) and adjusting orientation θ between the axis of ultrasound probes (5) and permanent magnets (6) inside the housing fixture (8).

10. The non-invasive magneto-acoustic apparatus as claimed in claim 1, for use in healthy tissue regeneration in damaged tissue treatment and also cancer tissue regression in cancer treatment.
